# EUROPEAN PATENT APPLICATION

(11) **EP 4 169 537 A1**
(43) Date of publication of application: **26.04.2023**
(21) Application number: 22202726.0
(22) Date of filing: 20.10.2022
(51) Int. Cl.: A61L 9/20, F24F 8/22, F28D 1/00

(54) **DEVICE FOR THE FLUIDS SANITIZATION AND RELATIVE SANITIZATION METHOD**

(30) Priority: 20.10.2021 IT 202100026906
(71) Applicant: Fantini Cosmi S.p.A., 20090 Settala (Milano) (IT)
(72) Inventor: BRAMBILLA FANTINI, FRANCO, Milano (IT); LA CAPRUCCIA, ROBERTO, Milano (IT)
(74) Representative: Mazzitelli, Maria

(57) **Abstract**

A description is given of a device (1) for sanitizing fluids comprising a fan (5) actuated by a motor and housed in a tubular conduit (10) in which fluid is propelled and a heat exchanger (7) in the form of a ceramic grid recuperator placed downstream of the fan (5), wherein the surfaces of the ceramic heat exchanger (7) impacted by the fluid are coated with photocatalytic material and associated with said exchanger (7) are a first lighting means (8) and a second lighting means (9) with LEDs, both of which are ultraviolet type A (UV-A), suitable for illuminating the surfaces coated with photocatalytic material.

## Description

The present invention relates to a device for the sanitization of fluids by exploiting the combined phenomenon of photocatalysis and the virucidal action of ultraviolet rays.

### Background of the invention

Air sanitizers are devices that have already been on the market for years. Sanitization is that process whereby harmful or noxious elements such as mould, bacteria, viruses, etc. are eliminated or rendered harmless.

One of the typical areas of application for sanitization devices is represented by the interior spaces of buildings, particularly residential buildings. With recent regulatory requirements and the consequent development of building techniques and materials, the energy requirement of buildings has been drastically reduced. The side effect of this important advantage is the near absence of natural ventilation which, if not appropriately compensated for with a mechanical ventilation system, makes any building/space unhealthy within a few hours of occupancy.

Such ventilation devices substantially comprise a fan housed in a tube, actuated by a motor mounted in a support frame provided with a circular hole for the passage of the fluid. Normally, in these systems, a ceramic recuperator is also provided whose function is to recover part of the heat inside the home. With the installation of such a system, the constant renewal of air in the rooms is guaranteed, at the same time extracting the stale and polluted air, more than 90% of whose thermal energy is reused to preheat the fresh air.

Such ventilation systems, however, do not guarantee optimal levels of sanitization, which has also become of utmost importance in light of the recent pandemic.

There are several methods of achieving sanitization. A first method is to use generators of ozone, which is a molecule composed of three oxygen atoms. Ozone is an unstable gas with an oxidizing effect that neutralises organic matter when it comes into contact with it. It is therefore able to oxidize and consequently destroy bacteria, viruses and other germs, but its oxidizing effect is not limited however to the environment: if it is present in too high a concentration, it can also be harmful to the human organism.

A second method is by saturation with chemical substances. This type of environmental sanitizer exploits the properties of certain chemical substances (which may be synthetic or natural) to sanitize environments by evaporating (through a device or naturally) until the environment is saturated.

Sanitization can also be achieved through ionisers. The electrically charged and released ions join the particles suspended in the air, charging them electrically and making them adhere, by electrostatic attraction, to the elements in the room (walls, floors, furniture, etc.). In this way, the particles are no longer free in suspension.

A further method of sanitizing is by means of high temperature: the air that comes into contact with a material at high temperature achieves the destruction of the particles present.

The methods described hitherto all have disadvantages. Ozone is considered harmful and therefore highly inadvisable on health grounds. Ionisers also have the disadvantage that they produce ozone by using the high voltages necessary to generate the ions. The drawback of using chemical substances is the use of very volatile compounds and therefore with evaporation in a short time. They also require daily maintenance, have ongoing costs and are not without side effects and allergic sensitization to the chemicals used.

The use of high temperature involves extremely long sanitization times (the air reaches the sanitizing surface without fans, taking advantage of the recirculation due to the hot air that tends to rise), while for the use of ultraviolet rays, attention must be paid to the escape of ultraviolet light from the device, which is carcinogenic. Furthermore, there are high costs in that these devices use lamps which, in order to be effective, need to be replaced very frequently. Finally, in order to achieve the sanitizing effect, the particles must be subjected to the action of the ultraviolet light for a long period, which is not always compatible with the times of transit inside the sanitizer.

Sanitizing by mechanical action, on the other hand, is very difficult to achieve, due to the minute size of the particles to be blocked.

In addition to the systems just described, devices have recently been developed that exploit photocatalysis for sanitization purposes. Photocatalysis is a natural phenomenon in which a nanomaterial substance, known as a photocatalyst, through the action of light - natural or artificial - gives rise to a chemical reaction involving the production of radicals, i.e. oxidizing substances capable of attacking the defences of pathogens and breaking them down at a molecular level, the end result of this process being exclusively non-harmful products such as water vapour, carbon dioxide, calcium carbonate, etc.

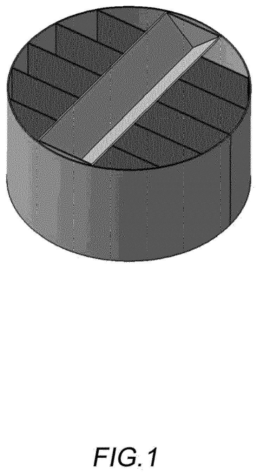

The state of the art for the use of photocatalytic surfaces, obtained by means of the application of a layer of photocatalyst nanomaterial, requires the air (or water in the case of water purification) to pass through and come into contact with the active surface. The larger the surface area available, the more fluid that will come into contact and thus the greater the sanitizing effect. It is also required that the photocatalytic surfaces be suitably illuminated in order to perform their function. To achieve this, standard sanitization systems typically make use of a structure like the one in Figure 1, consisting of a series of parallel surfaces between which the fluid flows and a support on which the illuminating elements are placed.

This approach has a series of disadvantages. The first is that it requires a dedicated structure and therefore with additional cost. The second is that the structure is cumbersome and is more cumbersome the more the surface area is increased. The third is that power must be carried to the lighting device in an area where the fluid flows and typically there are no cable runs.

Of the various photocatalysts, the most commonly used substance is titanium dioxide (TiO₂), chosen for its non-toxicity, cost-effectiveness, availability and photochemical stability, as it is a semiconducting oxide with a high reactivity so that it can be chemically activated by light. TiO₂ occurs in nature in various polymorphic forms, the main ones being anatase (tetragonal), rutile (tetragonal) and brookite (ortho-rhombic). Although rutile is the most stable phase, the polymorph most often used in photocatalysis is anatase, for reasons that will be discussed in greater detail below.

When titanium dioxide is exposed to sunlight or to an artificial light source, it absorbs ultraviolet (UV-A) radiation. Absorption of a sufficiently energetic photon by an electron in the valence band of titanium dioxide excites the electron itself, and promotes it into the conduction band, giving rise to the electron (e-) and gap (h+) pair. The process ends with the release of hydroxyl radicals, superoxide ions and hydroperoxide radicals, which are highly reactive substances capable of oxidizing the organic material present in the environment

The strong oxidative effect created by light, oxygen, water and titanium dioxide thus leads to the decomposition and transformation of bacteria, viruses and fungi into harmless substances. Photocatalytic surfaces therefore prevent the growth of microorganisms and do not allow the accumulation of the substances on which these microorganisms feed. It is therefore found that the presence of oxygen and water molecules in the reaction environment is essential. Indeed, their presence leads to the formation of highly reactive species, which contribute to improving the activity of the TiO₂ photocatalyst in the degradation of reaction volatile organic compounds (VOCs).

As mentioned, titanium dioxide expresses maximum potential when exposed to a source of ultraviolet light. It should be remembered at this point that UV mercury lamps for virucidal action are already widely used means of sterilisation. However, they have several critical factors. Firstly, UV lamps are fragile and therefore present a risk of mercury escaping through breakage when subjected to shocks. Secondly, the heating time is long. Various studies also show that it cannot exhibit maximum effectiveness at low temperatures. Because of these critical issues, UV light-emitting diode (UV-LED) technology has recently been developed as an alternative to the use of mercury lamps.

A further working possibility is the use of UV-C rays with a wavelength of 253.7 nm, which enables the destruction of the DNA (cell structure) of viruses, bacteria, moulds, fungi and other microorganisms by preventing reproduction thereof.

### Summary of the invention

From the foregoing, the disadvantages of currently known solutions are clear.

The object of the present invention is therefore to provide a device for sanitizing fluids by exploiting the photocatalysis phenomenon capable of overcoming the disadvantages of the prior art.

More particularly, an object of the present invention is to provide a device for sanitizing fluids by means of photocatalysis capable of obtaining greater results in terms of sanitizing efficiency without radically altering the structure of the devices currently used.

A further object of the invention is to provide a sanitizing device capable of achieving high sanitizing efficiency even when working in a single pass, without the need for recirculation.

A further object of the present invention is to provide a sanitizing device capable of combining the action of photocatalysis and the virucidal and germicidal action of UV-C rays in order to increase the efficacy and sanitizing power.

Another object of the present invention is to present a particular internal aluminium structure of the disinfection chamber which provides additional refraction of UV-C radiation, thereby increasing the efficiency of the radiation, allowing the total exposure of the microorganisms to the effect of the UV-C radiation from all sides, ensuring effective and healthy disinfection of the fluid.

Yet another object of the present invention is to provide a sanitizing device that is inexpensive and simple to produce, at the same time being highly efficient, safe, durable and environmentally sustainable.

These and other objects are achieved by a device for sanitizing fluids having the features listed in the appended independent claim 1 and by a relative sanitizing method according to the independent claim 10.

Advantageous embodiments of the invention are disclosed by the dependent claims.

Substantially, the present invention relates to a device for sanitizing fluids comprising a fan actuated by a motor housed in a tubular conduit in which fluid is propelled, and a heat exchanger in the form of a ceramic grid recuperator placed downstream of the fan, in which the surfaces of the ceramic heat exchanger impacted by the fluid are coated with photocatalyst material and a first lighting means and a second lighting means by LEDs, both with type A ultraviolet rays, are associated with said heat exchanger, capable of illuminating the surfaces coated with photocatalyst material.

The invention likewise relates to a method for sanitizing fluids by means of photocatalytic surfaces activated by electromagnetic radiation.

### Brief description of the drawings

Further features of the invention will be made clearer by the following detailed description, referring to a purely illustrative, and therefore non-limiting, embodiment illustrated in the accompanying drawings, in which:
Figure 1 is a schematic perspective view of a device for sanitizing fluids according to the prior art;
Figure 2 is an axonometric view of the external appearance of an exemplary embodiment of a sanitizing device according to the present invention;
Figure 3 is an axonometric sectioned view of the sanitizing device of Figure 2;
Figure 4 is an axonometric view of a sanitizing and lighting assembly provided in the sanitizing device according to the invention;
Figure 5 is an axonometric view of a reflecting cylinder used in combination with a lighting system to be inserted in the conduit of the sanitizing device; and
Figure 6 is an explanatory schematic representation of the functioning of the sanitizing device according to the invention.

### Detailed description of the invention

A preferred embodiment of a sanitizing device according to the present invention will now be described in detail with reference to the accompanying drawings.

In the present invention, the aim is sanitization by means of ultraviolet ray light. High-energy electromagnetic radiation is in fact capable of causing a genetic alteration of the cells of microorganisms leading to their inactivation and death.

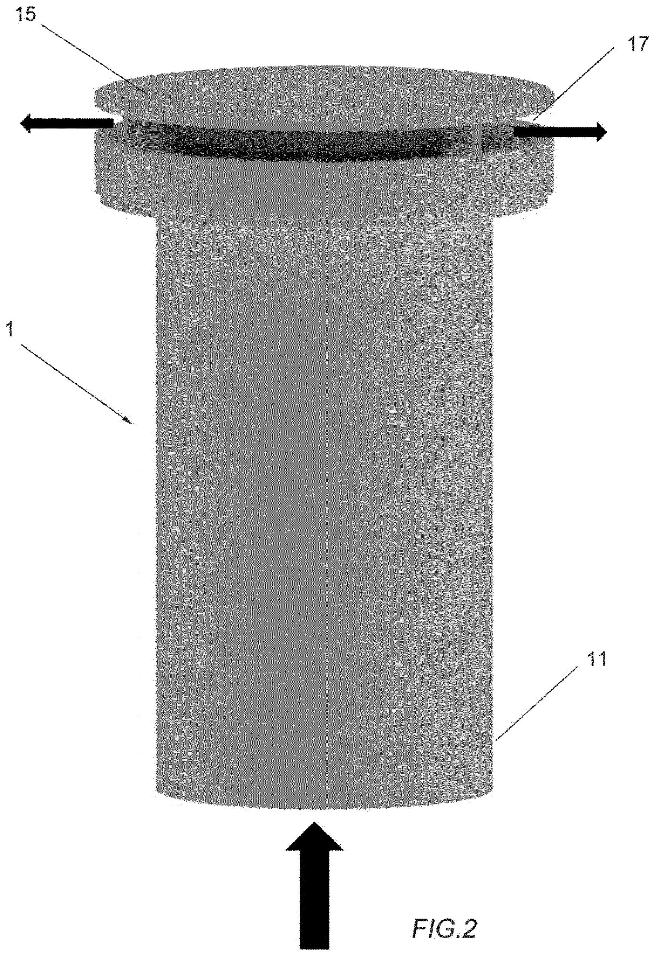

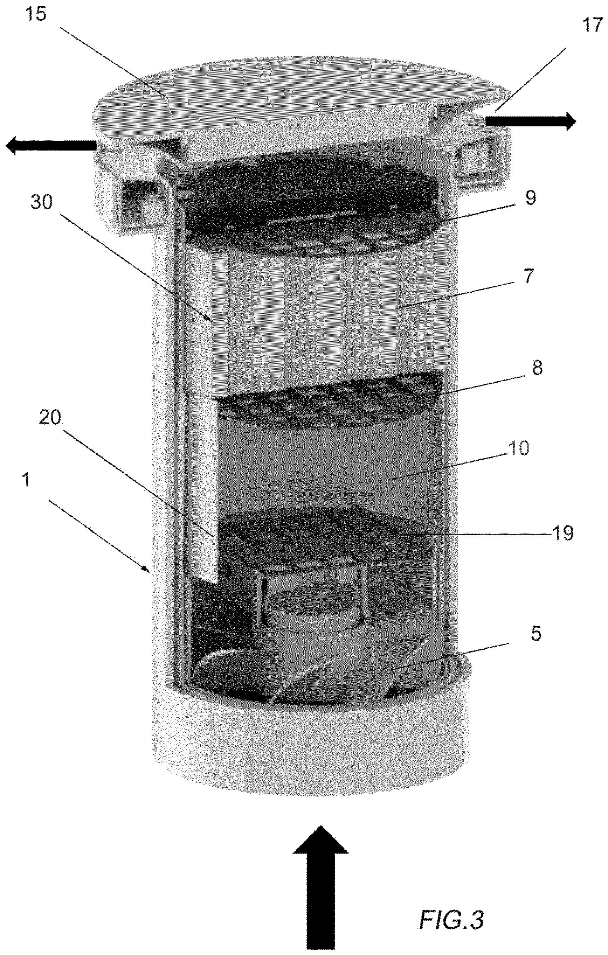

Figure 2 shows an exemplary embodiment of a sanitizing device 1, which is presented externally with a casing 11 of substantially cylindrical shape. Further details can be appreciated from the following drawings. Figure 3 is a cross section of the sanitizing device 1 of Fig. 2, in which a conduit 10 has been formed inside the casing 11 for the passage of fluid. The direction of the flow is indicated by the arrows in the drawing, with the fluid that enters through a particulate filter 3, passing through the conduit 10 and exiting, in this exemplary embodiment, through an annular slit 17 which is obtained after the application of a covering element, with a substantially aesthetic function. Clearly, the exiting of the fluid can also take place directly from the conduit or via any other means provided, this aspect not being a characterising part of the present invention.

The sanitizing device 1 according to the invention further comprises a fan 5 housed in the conduit 10 and actuated by a motor (not shown). Conventionally, the device also has a heat exchanger 7 in the form of a ceramic recuperator.

In the present invention, the ceramic recuperator 7 has been made photocatalytic, which normally, in these ventilation and sanitization systems, already has the function of recovering part of the heat inside the building. To become photocatalytic, the ceramic recuperator 7 is subjected to a process of deposition of a thin film of titanium dioxide (TiO₂), particularly on the surfaces over which the fluid flows. In operation, the photocatalytic surfaces are subjected to illumination by UV-A LED lamps. The band gap of anatase, which is the preferred form of TiO₂ for use as a photocatalyst, is in fact 3.2 eV. Radiation with a wavelength of less than 400 nm (near UV) is therefore required for its activation. This type of ultraviolet is not damaging and safe. As anticipated, the inactivation of bacteria by heterogeneous photocatalysis using UV-A (315-400 nm) and *TiO₂* is considered one of the most effective disinfection technologies, in that no carcinogenic, mutagenic or malodorous compounds are formed during the process.

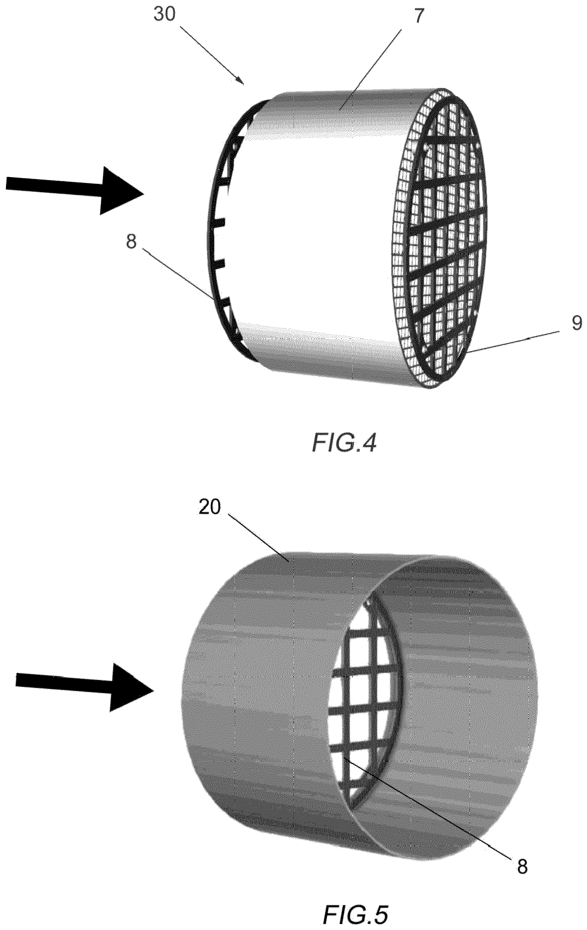

In the sanitization device of the present invention, the heat exchanger (ceramic recuperator) 7 is coupled to two different UV-A type LED lighting means 8 and 9 which are capable of illuminating the photocatalyst coated surfaces from two opposite directions. According to an advantageous aspect, the heat exchanger 7 can be coupled directly with respective LED lighting sources 8 and 9 in an assembly 30, shown separately and axonometrically in Figure 4. In this embodiment, the heat exchanger 7 is formed by a grid between which the fluid passes and whose surfaces are coated with titanium dioxide, and the lighting sources 8 and 9 are UV-A LED boards. In particular, LED boards 8 and 9 are discoidal in shape in order to couple with heat exchanger 7, which has a cylindrical shape.

Such a solution is particularly advantageous: no extra element is added to the system (the ceramic recuperator is already present) and therefore bulk and costs are reduced. In addition, it is not necessary to carry power cables for lighting in places not reached by such a power supply because the ceramic recuperator 7 can be placed in proximity of the motor, which is by its nature already powered.

The light can therefore be placed close to the motor, sharing its power supply if necessary. The ceramic recuperator also has in itself a large surface area available, allowing a large sanitizing area and thus realising effective sanitization on almost all the air passing through.

Room sanitization normally works by recirculation: a volume of air (or of water) to be sanitized passes through the sanitizing element several times in order to increase the effectiveness of the sanitization. The system according to the invention can work on a single pass, without the need for recirculation. In fact, laboratory tests have confirmed that effective sanitizing is achieved even with a single pass. This is because the ceramic recuperator 7 comes into contact with the fluid that is fed in and therefore the sanitizing action, which is instantaneous and acts immediately, is applied to almost its entire volume.

Advantageously, the sanitizing device according to the present invention also exploits the action of a C-type LED lighting medium, whose ultraviolet (UV-C) rays reach an optimal germicidal force near 253.7 nm. Since ozone can only be produced below 200 nm, the germicidal wavelength of UV-C does not generate ozone.

Titanium dioxide is a photosensitiser of particular interest as regards bacterial inactivation. Photogenerated free radicals or ROS (Reactive Oxygen Species) can attack the microorganism from outside, initially oxidizing the cell membrane (mainly by lipid peroxidation) and then destroying nucleic acids, proteins (deactivation of enzymes), etc. The ROS generated by the photocatalyst, which attack the molecule outside oxidizing it by lipid peroxidation, offer revolutionary chain oxidation-reduction characteristics and this increases the damage to the attacked molecule. The combined reaction of UV-C and ROS is even more aggressive, to the point of involving core proteins and breaking DNA molecular bonds. The damaged organism can no longer produce critical proteins or reproduce and quickly dies.

UV-C radiation is then absorbed by the DNA and by the RNA of microorganisms such as viruses and bacteria, changing their structure and hindering their reproduction. With a sufficiently powerful radiation intensity, UV-C is a very reliable, fast and environmentally friendly method of disinfection because it does not require the use of chemicals. According to an embodiment of the present invention, a UV-C radiation amplification system 20 is provided inside the conduit 10 of the device 1.

More particularly, the amplification system takes on the form of a reflecting cylinder 20 formed by a selective reflection aluminium tube which allows 91% of UV-C rays reflection to be reached, thus increasing the overall effectiveness of the system and therefore the disinfecting power. Figure 5 shows an example of a reflecting cylinder 20, which is normally provided in the conduit 10 coaxially to the axis of the fan 5, coupled to a UV-C LED board.

According to a preferred aspect of the present invention, the UV-C LED lighting is formed on the same structure 8 on which one of the UV-A LED boards is mounted in a top-bottom configuration, with the UV-A light turned towards the ceramic recuperator 7 and the UV-C light turned towards the fluid inlet, after undergoing the amplifying action of the reflecting cylinder 20.

Advantageously, according to the size of the device 1 and the length of the tubular conduit 10, a second lighting system 19 can be provided to emit UV-C type radiation, in particular a UV-C LED board, downstream of the fan 5 and preferably upstream of the UV-C lighting system 8, so as to amplify the virucidal action in consideration of the volume of fluid in input.

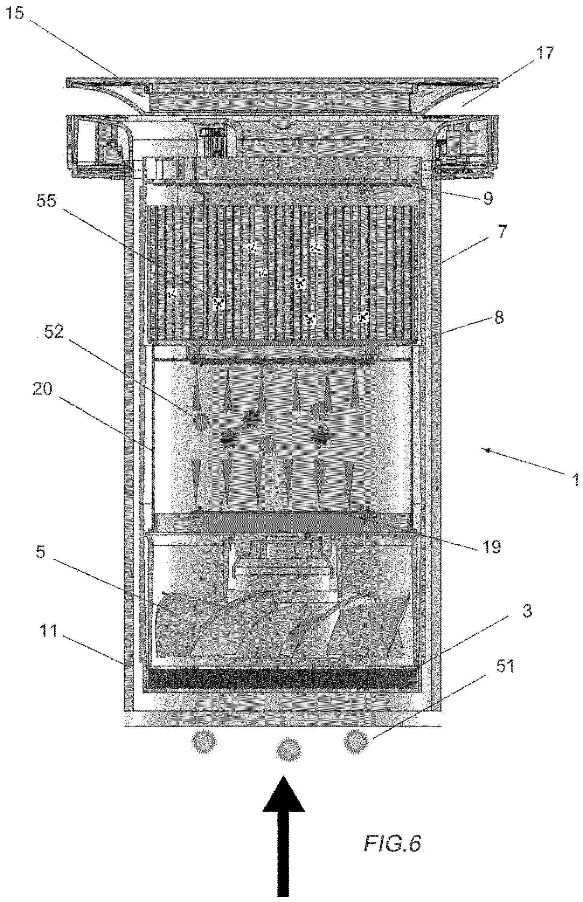

Figure 6 is a schematic representation of the functioning of a sanitizing device according to the latter preferred aspect of the invention and of a method for sanitizing fluids by means of photocatalytic surfaces activated by electromagnetic radiation. A fluid flow enters the device through a particulate filter 3, capable of filtering solid particulate matter 51 (PM 1.0, PM 2.75, PM 7.7, PM 10, PST). The filtered fluid is then set in motion by the propeller 5 and pushed into the conduit 10, carrying with it harmful substances such as viruses 52 and bacteria 53. In this area, the fluid undergoes its first germicidal and virucidal attack by the UV-C LED lights arranged on one side of the first lighting means 8, and optionally on the second UV-C lighting system 19, whose action is amplified by the presence of the reflecting cylinder 20.

The fluid then enters the heat exchanger 7, the surfaces of which have been coated with titanium dioxide and on which UV-A LED rays are emitted by the first and second lighting means (8, 9). In this area, the combined action of UV-A and TiO₂ has an effect in particular on volatile organic compounds (VOCs) 55 such as CO₂, CO and NO. In output from the sanitizer, there is thus a flow free of bacteria, viruses, VOCs and particulate matter.

A device described in this way has numerous advantages with respect to known solutions, including:
- Cost-effectiveness: the invention does not restrict the operation of the environment nor is the production cycle interrupted during sanitization. In addition, there is no need for personnel to intervene.
- Durability: the photocatalytic reaction does not consume the catalyst (titanium dioxide), therefore there is no need to replace the module and this ensures a lifespan equal to the life of the substrate in which it is inserted.
- Rapidity: it has been demonstrated that the system is able to eliminate harmful microorganisms after just a few minutes.
- Eco-sustainability: with the system according to the invention there is no dispersion of pollutant material, so the environmental impact is zero. The surfaces are safe to touch and do not release dust. The low energy consumption of the LEDs used allows for greater respect for nature.

Naturally, the present invention is not limited to the particular embodiments previously described and illustrated in the accompanying drawings, but numerous detailed changes may be made thereto, within the reach of the person skilled in the art, without thereby departing from the scope of the same invention, as defined by the appended claims.

## Claims

1. Device (1) for sanitizing fluids comprising a fan (5) actuated by a motor and housed in a tubular conduit (10) in which fluid is caused to flow and a heat exchanger (7) in the form of a ceramic grid recuperator placed downstream of the fan (5), **characterised in that**
the surfaces of the heat exchanger (7) impacted by the fluid are coated with photocatalytic material and with said heat exchanger (7) are associated a first LED lighting means (8) and a second LED lighting means (9), both of type A ultraviolet rays (UV-A) suitable for illuminating surfaces coated with photocatalyst material.

2. Device (1) for sanitizing fluids according to claim 1, **characterised in that** said heat exchanger (7) has a cylindrical shape and said first and second lighting means (8) and (9) are discoid-shaped type A ultraviolet (UV-A) LED boards placed upstream and downstream of the heat exchange (7) respectively.

3. Device (1) for sanitizing fluids according to claim 2, wherein said heat exchanger (7) and said lighting means (8, 9) are integrated into an assembly (30).

4. Device (1) for sanitizing fluids according to any one of the preceding claims, **characterised in that** the photocatalytic material with which the surfaces of the heat exchanger (7) are coated is titanium dioxide (TiO₂), preferably in anatase form.

5. Device (1) for sanitizing fluids according to any one of the preceding claims, further comprising a type C ultraviolet (UV-C) lighting means provided upstream of the heat exchanger (7) suitable for emitting UV-C rays towards the incoming fluid.

6. Device (1) for sanitizing fluids according to claim 5, **characterised in that** said UV-C type lighting means shares the same structure as the first UV-A type lighting means (8), having a UV-A type LED board on the side turned towards the heat exchanger (7) and a UV-C type LED board on the side turned towards the fluid inlet.

7. Device (1) for sanitizing fluids according to claim 5 or 6, wherein said UV-C lighting means is associated with a UV-C ray amplification system (20).

8. Device (1) for sanitizing fluids according to claim 7, **characterised in that** said UV-C ray amplification system (20) is made up of a reflecting cylinder formed by a selective reflection aluminium tube mounted internally and coaxially to the conduit (10).

9. Device (1) for sanitizing fluids according to any one of claims 5 to 8, comprising an additional UV-C LED board (19) provided downstream of the fan (5) suitable for emitting UV-C rays towards the incoming fluid.

10. Method for sanitizing fluids by means of photocatalytic surfaces activated by electromagnetic radiation, consisting in sending the fluid to be sanitized by means of a fan (5) into a tubular conduit (10) comprising a heat exchanger (7) in the form of a ceramic grid recuperator placed downstream of the fan (5), **characterised in that** the surfaces of the heat exchanger (7) impacted by the fluid are coated with photocatalytic material and that, in operation, they are illuminated from two directions by a first lighting means (8) and by a second lighting means (9) with LEDs, both of type A ultraviolet (UV-A).

11. Method for sanitizing fluids by means of photocatalytic surfaces activated by electromagnetic radiation according to claim 10, further comprising the illumination of the incoming fluid by type C ultraviolet (UV-C) radiation by means of a UV-C lighting system provided upstream of the heat exchanger (7).
